# EUROPEAN PATENT APPLICATION

(11) **EP 4 778 548 A1**
(43) Date of publication of application: **22.07.2026**
(21) Application number: 25153145.5
(22) Date of filing: 21.01.2025
(51) Int. Cl.: A61M 5/14, A61M 39/22, A61J 3/00, B01F 33/84, B01F 35/71, F16K 11/00

(54) **MULTI-WAY VALVE DEVICE**

(71) Applicant: Lizenzia GmbH, 3645 Gwatt (CH)
(72) Inventor: GYGER, Fritz, 3645 Gwatt (DE)
(74) Representative: Keller Schneider Patent- und Markenanwälte AG

(57) **Abstract**

A multi-way valve device (100) comprising a valve housing (101) accommodating at least a first valve (110) and a second valve (140), wherein the first valve (110) comprises a first valve inlet (111) and a first valve outlet and the second valve (140) comprises a second valve inlet (141) and a second valve outlet and the valve housing (101) comprises a unifying channel (102), wherein the first outlet and the second outlet lead into the unifying channel (102).

## Description

### Technical Field

The invention relates to a multi-way valve device comprising a valve housing accommodating at least a first valve and a second valve, wherein the first valve comprises a first valve inlet and a first valve outlet and the second valve comprises a second valve inlet and a second valve outlet. The invention further relates to an arrangement comprising a first multi-way valve device and a second multi-way valve device, to a method for cleaning such an arrangement, to a method for controlling a multi-way valve device and to uses of a multi-way valve device.

### Background Art

In chemical laboratories and in medical applications, the use of valves and multi-way valves in particular is ubiquitous. Multi-way valves are essential components for managing complex fluid pathways in analytical and experimental setups as well as in medical applications. The use of multiway valves allows for particularly fast switching between different fluids. In analytics, for example, different solvents can be selected. In medical applications, for example, different medications can be selected for an infusion.

In medical applications, the precise dosing of small volumes is of particular interest, especially when the liquids to be dosed are medications. In known systems, syringes are used whose plungers are driven by a motor to dispense the liquid. The dosage amount is controlled by the plunger's advance path, and the volume flow is controlled by the plunger's advance speed. Such devices are well known and are used, for example, in intensive care units. However, these devices are not very precise.

The known multi-way valves have the disadvantage that they do not work very precisely and/or have insufficient repeatability. Thus, precise dosing of the fluid cannot be guaranteed.

### Summary of the invention

The object of the invention is to create a multi-way valve device belonging to the technical field initially mentioned, which is designed in such a way that a particularly precise dosage of a fluid can be achieved and which is particularly simple in design.

The solution to the problem is specified by a device according to the features of independent claim 1. According to the Invention, the valve housing comprises a unifying channel, wherein the first outlet and the second outlet lead into the unifying channel.

The individual outlets of the first valve and the second valve are combined by the unifying channel. This means that different media can be fed through the unifying channel and, if necessary, mixed in the unifying channel. Since the unifying channel is part of the multi-way valve device, the paths between the first valve or the second valve and the unifying channel can be kept particularly short, thus enabling precise dosing. If, for example, a carrier liquid is fed via the first valve and a reagent is added via the second valve, the start and end point of the carrier liquid containing the reagent can be precisely controlled.

The multi-way valve device comprises a valve housing, which includes at least a first and a second valve. The multi-way valve device thus forms a unit as a whole. The valve housing and the first and second valves are fixed relative to one another so that no relative movement occurs between the valve housing and the first or second valve when the multi-way valve device is handled. Thus, the multi-way valve device differs significantly from conventional arrangements of several valves, which are loosely connected to each other via hoses and hose connectors (such as T-pieces and the like).

The first valve and the second valve each comprise a valve inlet and a valve outlet. The first valve comprises a first valve inlet and a first valve outlet, the second valve comprises a second valve inlet and a second valve outlet. The valve seat is arranged between the valve inlet and the valve outlet, which either opens or closes a passage for a fluid between the valve inlet and the associated valve outlet. In the preferred embodiment, the first valve and the second valve are designed in such a way that only an open state and a closed state can be set - it is therefore preferably a switching valve. In variants, however, the valve can also be adjustable in such a way that a half-open state can also be set. Furthermore, the first valve and the second valve can also be continuously adjustable.

To enable precise dosing with the switching valve, the switching cycles of the first valve and the second valve are preferably time-controlled. In some cases, the time control can also be dispensed with, especially if, for example, a volume flow is measured before the valve inlet, in the valve itself or after the valve outlet.

In the timing control regime, the first valve or the second valve is opened during a certain time interval in order to control the dosing volume. This system works perfectly as long as the pressure of the fluid to be dosed is sufficiently constant. Preferably, the liquid to be dispensed is pressurised with compressed air. However, this can be achieved also by various other means, for example, by using a dosing pump that maintains a constant pressure. Furthermore, the liquid level of a fluid container connected to the valve inlet can be kept constant. Experts are aware of further possibilities.

However, even if the fluid pressure is constant, sufficiently precise dispensing cannot be guaranteed in every case. Due to various influences, the resistance in the dispensing system as a whole can change, which also changes the flow rate and thus affects the precision of the dispensing. Such influences may be the temperature, which affects the viscosity of the fluid, but also the thermal expansion of the cross-sections in the lines and thus the flow resistance. In medical applications, such influences may also be present due to a kink in a tube, in a tube length, etc. The expert is aware of further influences that can affect precise dosing of the fluid.

What these factors have in common is that they change the pressure in the lines, which means that the dispensed volume also changes if time control is used.

Therefore, preferably a sensor, in particular a pressure sensor, is coupled to the unifying channel. This means that influences of the first and second valve can also be detected particularly well, in order to optimise the timing of the valves and thus improve the precision of the dosages.

With the arrangement of the sensor at the unifying channel, it is also achieved that exactly one sensor, in particular a pressure sensor, is sufficient for optimised control of the multi-way valve device. Therefore, in a preferred embodiment, the multi-way valve device comprises exactly one sensor, in particular exactly one pressure sensor. With exactly one sensor, the measuring device can be designed to be particularly simple and compact. This also makes a particularly cost-effective measuring device possible.

The pressure sensor can thus be integrated in the multi-way valve device. However, this is not absolutely necessary, the pressure sensor can also be arranged partially or fully outside of the multiway valve device, in particular between the multi-way valve device and a dispensing head or suction head. To do this, the pressure sensor can be arranged in a fluid line, in a hose, etc. The pressure sensor is used to measure pressure differences in front of the dispensing head/suction head in order to take into account, for example, level differences in the fluid containers and/or different installation heights of the fluid containers by correcting the opening times of the valves accordingly.

However, it is clear to those skilled in the art that more than one sensor can also be provided, which are not only arranged at the unifying channel, but also, for example, at the inlet or outlet of one of the valves. Different sensors can also be provided, in particular temperature sensors, flow sensors, bubble sensors, etc.

The bubble sensor can be of particular benefit in medical applications of the multi-way valve device in order to avoid air injections during infusions.

The bubble sensor, for example, can be designed as an ultrasonic sensor. Alternatively, an optical sensor can be used to detect bubbles. Such sensors are known to those skilled in the art.

The flow sensor can in turn be helpful to monitor the function of the multi-way valve device, i.e. to check whether the fluid flows through the multi-way valve device when a valve is open or does not flow when all valves are closed. The data from the flow sensor can also be used to control the dosing of the fluid or to control it more precisely together with other data, in particular data from a pressure sensor.

Instead of being an integral part of the multi-way valve device, one or more sensors can also be provided as a separate part from the multi-way valve device. In a preferred embodiment, a pressure sensor is coupled to the unifying channel, while the bubble sensor is coupled as a separate part from the multi-way valve device to a fluid line that connects to the unifying channel. Further variants are known to those skilled in the art. In some variants, the sensors can be dispensed with.

Preferably, the sensor is arranged lateral to the unifying channel, more preferably at right angles to a main flow direction of the multi-way valve device. The term 'main flow direction' is to be understood here as the averaged direction between the individual flow directions of the valves. If the valves are arranged in parallel, the 'main flow direction' corresponds to the direction of one of these valves. If the valves are arranged in pairs in a V-shape, the main flow direction runs through the centre of the V-shape.

The lateral arrangement of the sensor, in particular the pressure sensor, has the advantage that the sensor remains particularly accessible in the multi-way valve device. On the one hand, this simplifies the connection of the sensor, which also simplifies the construction of the measuring unit. Furthermore, it also simplifies the replacement of the sensor if necessary. During operation, this arrangement has the advantage that the sensor is not directly exposed to the flow, which means that particularly precise measurement data can be achieved. However, the sensor can also be arranged differently in variants.

The sensor is preferably aligned at right angles to the main flow direction. The alignment of the sensor refers to the direction of force in which the pressure force is measured. However, the sensor can also be aligned differently. The sensor can also be designed in such a way that the pressure can be measured from different sides of the sensor, for example to compensate for any pressure changes due to flow effects.

For example, the sensor can also be arranged in a bypass channel through which a partial flow of the fluid is diverted for measurement and then reunited with the main flow. Furthermore, the sensor can be attached directly to the unifying channel.

Preferably, the first valve and/or the second valve are designed as solenoid valves. This results in a valve that switches particularly quickly and precisely. Furthermore, solenoid valves can be designed to be particularly compact. After all, they have a simple design and are essentially maintenance-free. Alternatively, piezo valves, bimetallic valves, etc. can be used. Experts are aware of further variants.

Preferably, the first valve and the second valve are arranged in parallel. The parallel arrangement of the valves in the multi-way valve has the advantage that the multi-way valve can be designed to be particularly compact. In this case, the valves are preferably arranged next to each other.

In variants, however, the valves can also be arranged differently, in particular, the valve outlets can be arranged closer together than the valve inlets. In particular, for example, the valves can be arranged in pairs in a V-shape, with the valve outlets being closer together than the valve inlets. This arrangement has the advantage that the valve inlets are more accessible. This arrangement can be particularly advantageous in an application in which the connections to the valve inlets are regularly changed (e.g. when changing syringes). Furthermore, this arrangement has the advantage that the unifying channel can be made particularly compact. Finally, a person skilled in the art will also be aware of other possible arrangements.

Preferably, the multi-way valve includes at least two, preferably three, four, five or six valves. However, the multi-way valve can also include more than six valves, for example seven, eight, nine or more valves.

Preferably, the valves are arranged symmetrically to one another. For example, the valves can be arranged in the corners of a regular polygon. In a further possible arrangement, an additional valve can be arranged in the centre of the polygon. This allows optimal access to the individual valves while maintaining a compact design of the multi-way valve. In a further embodiment, the valves can also be arranged in a row. A person skilled in the art will be aware of further possible arrangements of the valves.

Preferably, the first fluid outlet and/or the second fluid outlet are connected to the unifying channel via a rigid fluid line. This enables a particularly compact design of the multiway valve. It is particularly preferred that the connections between the fluid outlets and the unifying channel are formed as a bore in the valve housing. Furthermore, the connections can also be formed as pipe connections.

In variants, the first fluid outlet and the second fluid outlet can also be connected to the unifying channel via a hose connection.

Preferably, the unifying channel is formed as bores in the valve housing. This allows a particularly compact design of the multi-way valve device. It is particularly preferred that the connecting channels between the valves and the unifying channel, as well as the unifying channel, are formed as bores in the valve housing. Alternatively, the unifying channel can be formed as a pipe or the like.

Preferably, the first valve and the second valve are designed as separate units from the valve housing, which can be connected to the valve housing in particular via detachable connections. This makes it particularly easy to replace the valves. Furthermore, this design simplifies the cleaning of the multiway valve device. If multi-way valve devices with different numbers of valves are provided, a particularly simple design can be achieved, since the same valves can be provided for each multi-way valve device. This results in a particularly simple and cost-effective design for multi-way valve devices with different numbers of valves.

In variants, the valves can also be fixed in the valve body so that they cannot be detached. The valves can also be formed in the valve body.

The valves are preferably designed to be cylindrical, at least in sections, so that they can be inserted into cylindrical bores in the valve body. This further simplifies the design of the multi-way valve device. The valves and corresponding seats in the valve body can also be designed differently.

Preferably, at least the first valve inlet comprises a first fitting for directly connecting the first valve inlet with a syringe, in particular a first Luer lock connection and, preferably, the unifying channel comprises a second fitting, in particular a second Luer lock connection. The Luer lock connection makes it particularly easy for the user to intuitively connect syringes to the multi-way valve device fluidic system. Luer lock connections are known to be safe, have a high level of leak-tightness - even under pressure - and are designed for sterile use. The Luer lock connection has the further advantage that it is established in the field of medicine and can therefore be used in a variety of ways. For example, a tube with a corresponding fitting or similar can be connected via the Luer lock connection instead of a syringe.

The fact that the syringe can be connected directly to the Luer lock connection of the valve makes it possible to achieve a particularly compact arrangement of the multi-way valve device with the syringes. In particular, this makes it possible to dispense with an additional attachment of the syringe to the multi-way valve device. On the other hand, the syringe can also be connected indirectly to the Luer lock of the valve via a tube or similar.

The syringes can be used as storage containers for the fluid. In medical applications, for example, the syringes can contain a saline solution for an infusion. Furthermore, the syringes can contain fluids for medical or therapeutic treatment.

In laboratory applications, in particular in chemical analysis, for example, the syringes can contain solvents, agents, etc.

Furthermore, the syringes may also contain cleaning fluids for cleaning the plunger. A person skilled in the art will be aware of further variants.

However, the valve inlet does not necessarily have to be equipped with a Luer lock connection. The skilled person is aware of other fittings for connecting tubes, syringes, pipes, etc., with which the valve inlet can be equipped.

Luer lock connections are particularly advantageous in medical applications, as they are a common connection in this field. For other applications, such as laboratory applications, these can also be used, but other connections with lower dead volumes are preferred there. Such connections can include tube connectors, flat-bottom fittings, coned port fittings, etc.

Preferably, all valve inlets are equipped with a Luer lock connection, in particular the first valve inlet and the second valve inlet. In variants, individual valve inlets can also be equipped with different fittings, i.e. the multi-way valve device can also be equipped with valves with different fitting.

In a particularly preferred embodiment, the multi-way valve device is arranged in use in such a way that a fluid flow is conveyed upwards through one or more of the valves against the force of gravity. This arrangement has the advantage that air bubble inclusions can be prevented more easily. It also makes it easier to connect an open syringe to a valve inlet, as the syringe opening can be directed upwards when connected to the fluid inlet.

In variants, the multi-way valve device can also be arranged in such a way that a fluid is conveyed downwards by gravity through one or more of the valves.

The invention further extends to an arrangement comprising a first multi-way valve device and a second multi-way valve device, wherein the first multi-way valve device and the second multi-way valve device are arranged in series, whereby the second multi-way valve device is arranged downstream of the first multi-way valve device.

This arrangement means that the unifying channel of the first multi-way valve device is fluidically connected to a fluid inlet of the second multi-way valve device. This arrangement means that more valves are available with which different fluid flows can be controlled. In laboratory applications, for example, different solvents, cleaning fluids, agents, etc. can be controlled. In medical applications, for example, more different medications, infusions etc. can be controlled. In variants, the multi-way valve device can also be used individually. Furthermore, the multi-way valve device can also be arranged in parallel or in more complex arrangements, both in parallel and in series.

In a first use, especially in a laboratory application, dosing medium and cleaning fluid are typically used. The dosing medium can be selected depending on the application. Non-exhaustive examples include acids, bases, buffer solutions, reagent solutions, organic solvents, etc. Different surfactant solutions, enzyme solutions, acids, bases, organic solvents or combinations thereof can be provided as a cleaning fluid. Other variants are known to the skilled person.

Preferably, a unifying channel of the first multi-way valve device is in fluid communication with an inlet of a first valve of the second multi-way valve device, an inlet of a first valve of the first multi-way valve device is directly connected to a source of a cleaning fluid, an inlet of a second valve of the first multi-way valve device is directly connected to a source of a dosing medium, and an inlet of a second valve of the second multi-way valve device is directly connected to the source of the dosing medium.

The term 'directly connected' is understood here to mean a connection that comprises a hose, a pipe, a channel or a combination thereof that is valve-free. Thus, when directly connected, the connection preferably comprises exclusively a hose, a tube, a channel or a combination thereof.

With this arrangement, it is now possible to switch automatically and in particular quickly between cleaning fluid and dosing medium. This results in a particularly efficient arrangement, especially when used in laboratory applications.

Preferably, a unifying channel of the second multi-way valve device is in fluid communication with a dispensing head, wherein the dispensing head preferably comprises at least two dispensing needles for dispensing the dosing medium.

In further embodiments, the dispensing head may also be designed with exactly one needle or without a needle, for example, merely with a spout or the like.

The dispensing head can be used to dispense a dosing medium into several wells, for example of a multiwall plate or into several test tubes or tubes. This means that the multi-way valve device can be used in a dosing system, for example in a Certus Flex^{®} system from Fritz Gyger AG.

In a preferred embodiment, a unifying channel of the first multi-way valve device is in fluid communication with an inlet of a first valve of the second multi-way valve device and an inlet of a first valve of the first multi-way valve device is directly connected to a source of a first medical fluid.

This allows the multi-way valve device to be used for the infusion of medical fluids. In particular, the multi-way valve device can be attached to an infusion stand in order to administer an infusion of medical fluids to a patient. In this form, the multi-way valve device can be used in hospitals, doctors' surgeries, rehabilitation centres, etc., but also in private homes. The source of a first medical fluid can include, for example, bags, bottles, ampoules, syringes or the like. These are preferably connected to the inlet of the first valve of the first multi-way valve device via hoses. Alternatively, other fluid connections can also be provided, such as tubes, etc.

Preferably, an inlet of a second valve of the first multi-way valve device is directly connected to a source of an infusion fluid, and an inlet of a second valve of the second multi-way valve device is directly connected to the source of a second medical fluid.

The arrangement can thus be used to mix a first medical fluid with the infusion fluid and inject it into a patient's body. Typical applications in medicine can thus be covered with the multi-way valve device respective with the arrangement comprising at least two multi-way valve devices.

In variants, the multi-way valve device can also be used in other ways, for example in laboratory applications or other specialities in which precise control of small volumes is important.

Preferably, the first medical fluid is contained in a first syringe, which is connected directly to the inlet of the first valve of the first multi-way valve device, preferably via a Luer lock connection and, preferably, the second medical fluid is contained in a second syringe, which is connected directly to the inlet of the first valve of the second multi-way valve device, preferably via a Luer lock connection.

This allows connecting the inlet of the multi-way valve device to a source of medical fluids particularly quickly and easily. Preferably, the inlet of the first valve of the first multi-way valve device comprises a Luer lock connection so that the syringe can be connected directly to the inlet. This allows the syringe to be essentially fixed relative to the multi-way valve device via the Luer lock connection (especially since no flexible connection, such as a tube or the like, needs to be provided between the syringe and the multi-way valve device). This means that the dead volume of the application can be kept particularly small. This in turn enables more precise dosing. The small dead volume also means that less medical fluid is required to fill the multi-way valve device (valves and channels of the multiway valve device, etc.). Finally, less material is contaminated, which would have to be cleaned or disposed of after use.

In variants, other sources for medical or other fluids can also be provided. In particular, the inlet of the valves can also be connected to hoses with various sources, especially, for example, to pumps that can pump fluids from containers that remain in place, or to bags that can transport the fluid to the valve by means of gravity, etc.

Preferably, the first syringe and the second syringe each comprise a plunger, which are pressurised at a constant pressure during operation.

The pressure can be built up in different ways. For example, a motorised drive can be provided to drive the plunger. The plunger can also be placed under constant pressure via a mechanical preload, in particular a spring. Pneumatic or hydraulic actuation of the plunger can also be provided in order to build up the pressure on the fluid. Finally, the plunger can also be pressurised by means of gravity, by applying a constant load to the plunger. Other possibilities are known to the skilled person.

Constant pressure on the plunger makes it particularly easy to control the multi-way valve device. This means that only a few influences need to be taken into account for the control in order to precisely control the volume to be dosed.

The skilled person is aware that, depending on the control system, the pressure applied to the plunger does not necessarily have to be constant. In particular, a pressure in the syringe can also be measured in order to incorporate this influence as precisely as possible into the control of the multi-way valve device.

Furthermore, a constant pressure can also be built up on the fluid without having to use a syringe. The fluid can also be supplied to the inlet via a pump, gravity or other means.

Preferably, a unifying channel of the second multi-way valve device is in fluid communication with a medical injection needle.

This means that the device can be used for injecting fluids into a patient. The unifying channel is particularly preferably connected directly to the injection needle. In variants, the injection needle can also be dispensed with, or a connection with an injection needle does not have to be made directly.

Preferably, a flow sensor and/or an air bubble sensor is coupled to the unifying channel of the first multi-way valve device. The flow sensor can be used to monitor the function of the multi-way valve device, i.e. to check whether the fluid flows through the multi-way valve device when a valve is open or does not flow when all valves are closed. The data from the flow sensor can also be used to control the dosing of the fluid or to control it more precisely together with other data, in particular data from a pressure sensor. Measurement errors can be prevented with the bubble sensor. Furthermore, the injection of bubbles into the body can be prevented, especially when administering infusions.

In both laboratory and medical applications, efficient and effective cleaning is of great importance after using the multi-way valve device or before changing the fluid. The multi-way valve device not only has the advantage that fluids can be dosed particularly precisely and that switching between different fluids can happen quickly, but the multi-way valve device can also be cleaned particularly quickly and effectively.

In a method for cleaning the above introduced arrangement, a cleaning fluid is successively fed through the first multi-way valve device and the second multi-way valve device. This allows the first and second multi-way valve device to be cleaned in succession with one cleaning fluid. This means that the multi-way valve device do not have to be cleaned separately, resulting in a particularly efficient cleaning process.

During the cleaning process, preferably a dosing medium or an infusion fluid is successively fed through the first multi-way valve device and the second multi-way valve device.

Thus, in the preferred method for cleaning the multi-way valve device, a dosing medium continues to be passed through the first multi-way valve device and the second multi-way valve device after the cleaning fluid has been passed through. The dosing medium is preferably the one that is to be used after the cleaning process. On the one hand, this cleaning process ensures that the first and second multi-way valve device no longer have any impurities from the previous use. On the other hand, it also ensures that neither cleaning agent nor a rinsing solution remains in the first and second multi-way valve device.

The cleaning agents used in the cleaning process are known to the skilled person and are generally matched to the type of contamination of the multi-way valve device.

In a method for controlling a multi-way valve device, data are measured, in particular pressure data of a dosing medium in the unifying channel, and a dosing volume of the dosing medium is controlled on the basis of the data.

The data are preferably used to control a dispensing valve, i.e. a dispensing valve, in particular a oneway valve, which is directly connected to the dispensing head for dosing the medium.

Alternatively, the data can also be used to control the multi-way valve device.

Preferably, the valves of the multi-way valve device, preferably all valves of the multi-way valve device, are switching valves with two states, namely open or closed. In the preferred embodiment, the flow rate through the multi-way valve device is thus controlled by opening or closing the respective valves. This results in a particularly simple and compact design of the multi-way valve device. In alternative embodiments, one or more of the valves can also be controllable in such a way that a flow rate can be set continuously.

In orderto control the flow rate, the pressure in the unifying channel is now preferably measured and one or more valves of the multi-way valve device are controlled on the basis of the measured pressure or values derived from the pressure and possibly other measured variables. In the particularly preferred embodiment, exclusively the measured pressure data is used to control the valves of the multi-way valve device.

Preferably, the valves of the multi-way valve device are time-controlled. This means that the pressure data determined with the pressure sensor of the unifying channel and the target value of the volume to be dispensed are used to calculate a time period during which the valve remains open. The time period can be adjusted dynamically based on the continuously measured pressure values. This allows external influences to be eliminated during dosing, which in turn enables particularly precise dosing.

Preferably, a fluid inlet of a first valve of the multi-way valve device is pressurised with the dosing medium at constant pressure. The process for controlling the flow rate can be further optimised if, the fluid to be dosed is at a constant pressure when it enters the valve inlet. This means that there is no need to measure the pressure at the inlet of the valve inlet, which results in particularly simple and efficient control of the multi-way valve device. Alternatively, the pressure at the valve inlet can also be measured and included in the control of the valves.

The multi-way valve device is preferably designed for volume flows lower than 10,000 nL/min, more preferably lowerthan 1000 nL/min, and particularly preferably lowerthan 100 nL/min. In variants, the valves can also be designed for volume flows higher than 10,000 nL/min.

Other advantageous embodiments and combinations of features come out from the detailed description below and the entirety of the claims.

### Brief description of the drawings

The drawings used to explain the embodiments show:
- Fig. 1a: a schematic cross-section through a multi-way valve device of a first embodiment with four valves aligned at the corners of a square, comprising a pressure sensor, along a plane parallel to the flow direction in the valves;
- Fig. 1b: a plan view of a multi-way valve device according to Figure 1a;
- Fig. 1c: a sectional view according to Figure 1a;
- Fig. 2: a schematic cross-section through a multi-way valve device according to figure 1a without pressure sensor;
- Fig. 3a: a schematic cross-section through a second embodiment of a multi-way valve device with three valves aligned at the corners of a equilateral triangle, along a plane parallel to the flow direction in the valves;
- Fig. 3b: a plan view of a multi-way valve device according to Figure 3a;
- Fig. 3c: a sectional view according to Figure 3a;
- Fig. 4a: a schematic cross-section through a third embodiment of a multi-way valve device with two valves, along a plane parallel to the flow direction in the valves;
- Fig. 4b: a plan view of a multi-way valve device according to Figure 4a;
- Fig. 4c: a sectional view according to Figure 4a;
- Fig. 5: a schematic cross-section through a multi-way valve device of a first alternative embodiment with four valves aligned in parallel, along a plane parallel to the flow direction in the valves;
- Fig. 6: a schematic representation of a first system using the third embodiment of the multiway valve device with two valves;
- Fig. 7a: a schematic representation of a second system using the first embodiment of the multi-way valve comprising a vacuum source and a fluid trap;
- Fig. 7b: a schematic representation of the second system according to figure 7a, wherein instead of a fluid trap, a volume for storing a fluid is provided in the periphery of the dispensing unit;
- Fig. 8: a schematic representation of a third system using three multi-way valves of the third embodiment;
- Fig. 9: a schematic representation of a fourth system using one multi-way valve of the first embodiment and two multi-way valves of the third embodiment; and
- Fig. 10: a schematic representation of a fifth system using three multi-way valves of the second embodiment.

In the figures, the same components are given the same reference symbols.

### Preferred embodiments

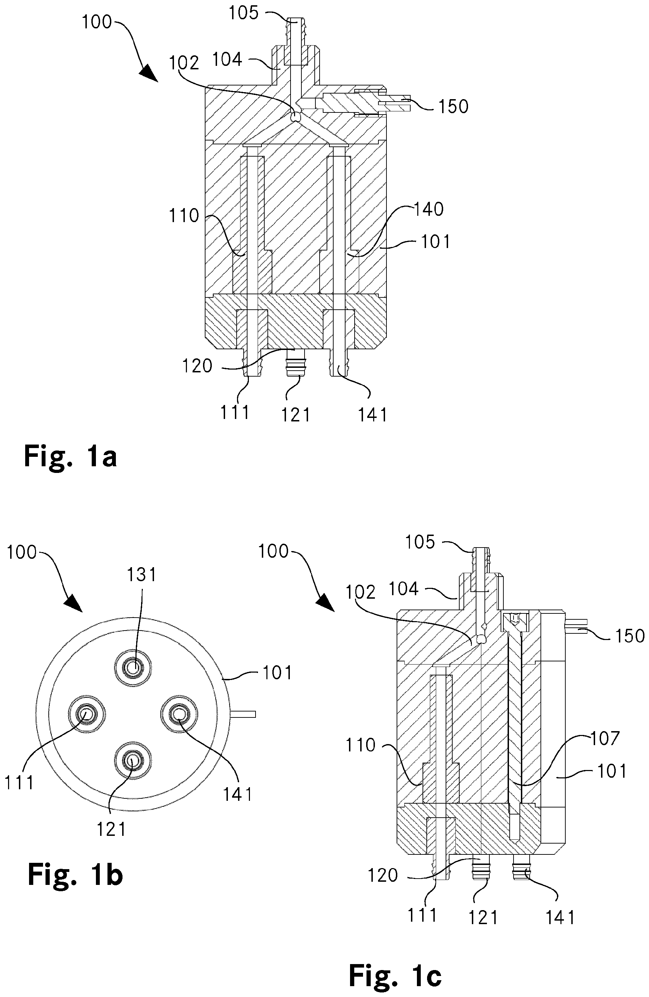

Figure 1 shows a schematic cross-section through a multi-way valve device of a first embodiment with four valves 110, 120 and 140 aligned at the corners of a square, along a plane parallel to the flow direction in the valves. Only the three valves 110, 120 and 140 are visible in the cross-section.

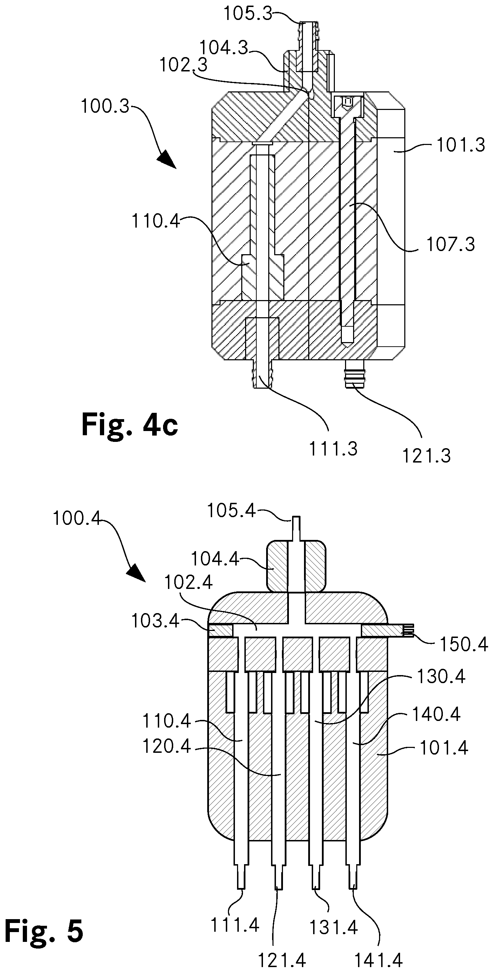

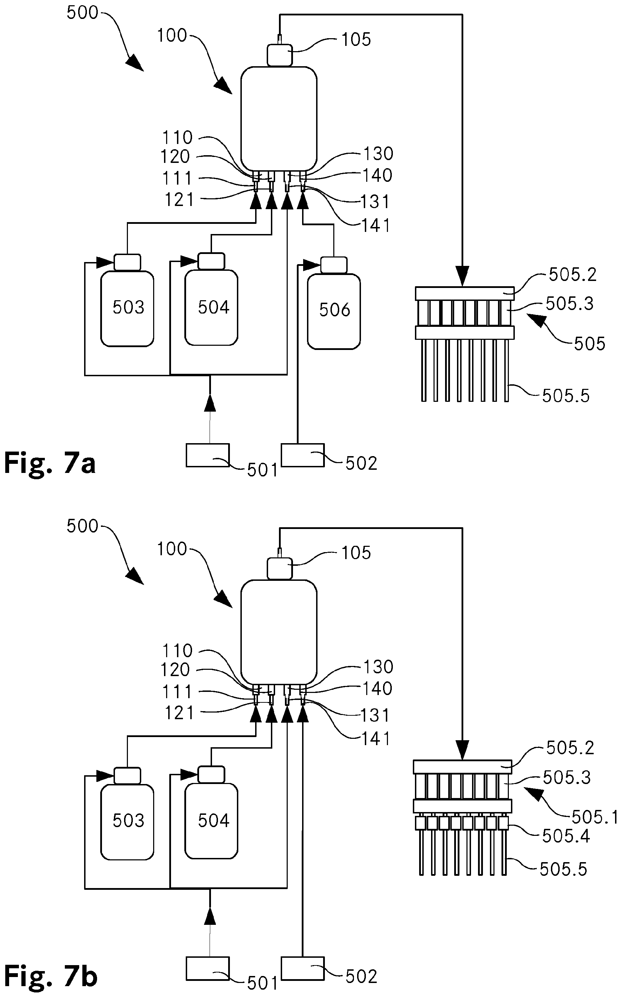

The four valves 110, 120 and 140 are accommodated in a valve housing 101. The valve housing 101 is essentially cylindrical in shape. The arrangement of the four valves 110, 120, 130 and 140 is shown in corners of a regular polygon, in particular in a square, so that adjacent valves are the same distance apart. Other arrangements are also possible, for example a polygonal arrangement with a centrally arranged valve, such an arrangement may be preferred in applications according to Figure 7 (see below) or the arrangement of the valves in a row (see figure 5 below). Each of the valves 110, 120 and 140 comprises a valve inlet, which is equipped with a fitting 111, 121 and 141. Hoses, syringes and the like can be attached to the fitting 111, 121 and 141 in a known manner.

The valve housing 101 further comprises a unifying channel 102 into which the valve outlets of the valves 110, 120 and 140 open. When, for example, valve 110 is open, a fluid can be conducted from the valve inlet 111 through the valve 110 into the unifying channel 102. The unifying channel 102 is aligned in parallel to the valve longitudinal directions. At right angle to the unifying channel 102, the unifying channel encompasses a pressure sensor 150, with which the pressure in the unifying channel 102 can be measured. In the process, the pressure sensor 150 can be used to monitor the pressure in order to control the valves 110, 120 and 140 based on the measured pressure data.

In this example, the pressure sensor 150 is integrated in the multi-way valve device. However, this is not absolutely necessary, the pressure sensor can also be arranged outside of the multi-way valve device, between the fitting 105 and a dispensing head or suction head. To do this, the pressure sensor 150 can be arranged in a fluid line, in a hose, etc.

The unifying channel 102 is ultimately connected to an output channel 104, which in turn is arranged in parallel to valves 110, 120 and 140. The output channel 104 finally opens into a fitting 105, via which the multi-way valve device can be connected to a dispensing head, an injection needle or another multi-way valve device.

Figure 1b shows a plan view of a multi-way valve device according to Figure 1a. Viewed from above, the arrangement of the four valves 110, 120, 130 and 140 can be seen in the corners of a square, based on the fittings 111, 121, 131 and 141.

Figure 1c shows a sectional view according to Figure 1a. The sectional cut-out covers an angle of 60°. In this illustration, it can be seen in the right-hand sectional view that the multi-way valve device is divided lengthwise into three parts, which are held together by screws 107.

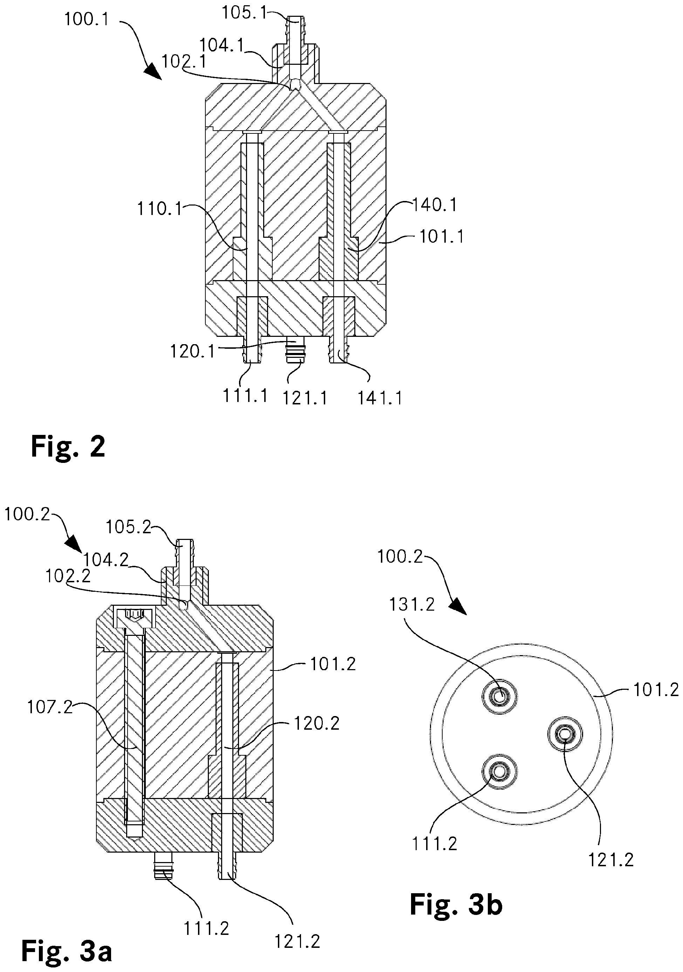

Figure 2 shows a schematic cross-section through a multi-way valve device 100.1 according to figure 1a without pressure sensor. For a simplified overview, the reference signs are each supplemented with '.1' in accordance with Figure 1a. In the application of this multi-way valve device, the pressure sensor is used outside of the multi-way valve device, for example in a connecting hose from the fitting 105.1 to a dosing head.

Figure 3a shows a schematic cross-section through a second embodiment of a multi-way valve device 100.2 with three valves 110.2, 120.2 and 130.2 aligned at the corners of an equilateral triangle, along a plane parallel to the flow direction in the valves.

Figure 3b shows a plan view of a multi-way valve device 100.2 according to Figure 3a, where fittings 111.2, 121.2 and 131.2 show the arrangement at the corners of an equilateral triangle.

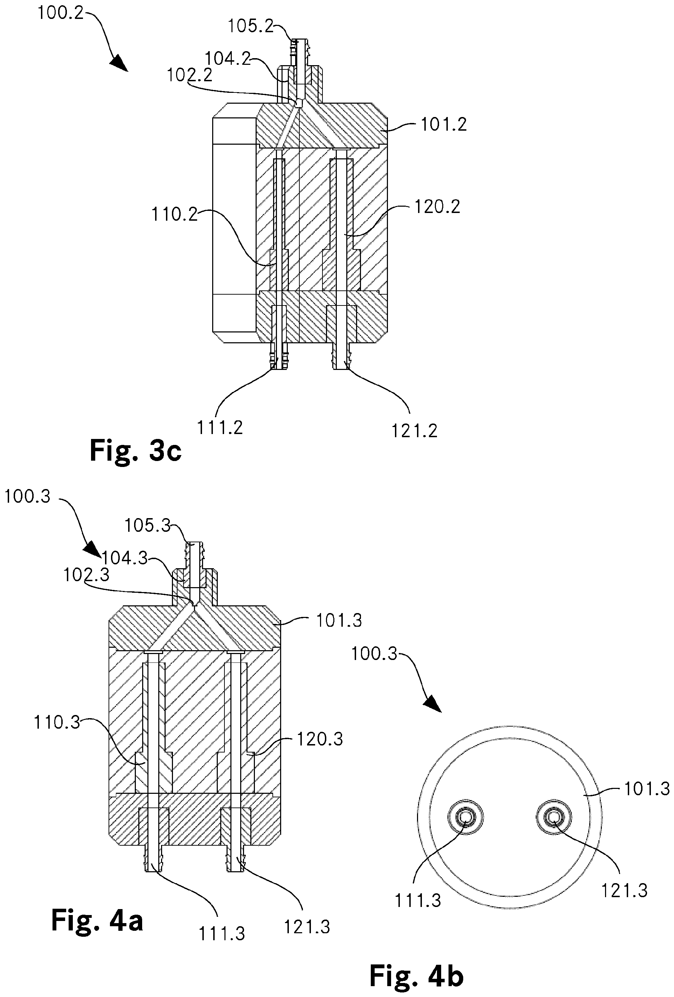

Figure 3c shows a sectional view according to Figure 3a. The sectional cut-out covers an angle of 60°. Therefore, the section in the present case runs through two valves 110.2 and 120.2. In this embodiment, the multi-way valve device 100.2 is also divided lengthwise into three parts, which are held together by screws (not shown).

Figure 4a shows a schematic cross-section through a third embodiment of a multi-way valve device 100.3 with two valves 110.3 and 120.3, along a plane parallel to the flow direction in the valves.

Figure 4b shows a plan view of a multi-way valve device 100.3 according to Figure 4a. The two fittings 111.3 and 121.3 are visible.

Figure 4c shows a sectional view according to Figure 4a. The sectional cut-out covers an angle of 60°. In this illustration, it can be seen in the right-hand sectional view that the multi-way valve device is divided lengthwise into three parts, which are held together by screws 107.3.

Figure 5 shows a schematic cross-section through a multi-way valve device of a fourth embodiment with four valves 110.4, 120.4, 130.4 and 140.4 aligned in parallel, along a plane parallel to the flow direction in the valves.

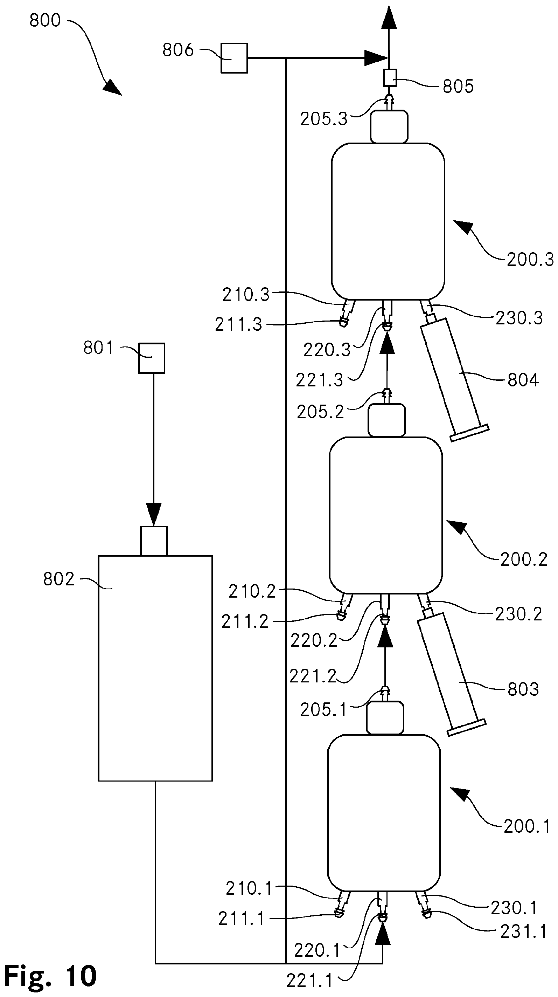

The four valves 110.4, 120.4, 130.4 and 140.4 are accommodated in a valve housing 101.4. The valve housing 101 is essentially cylindrical in shape. The arrangement of the four valves 110.4, 120.4, 130.4 and 140.4 is shown in a row in the present case, but they can also be arranged at the corners of a regular polygon, so that adjacent valves are the same distance apart. Other arrangements are also possible, for example a polygonal arrangement with a centrally arranged valve, such an arrangement may be preferred in applications according to Figure 10 (see below). Each of the valves 110.4, 120.4, 130.4 and 140.4 comprises a valve inlet, which is equipped with a fitting 111.4, 121.4, 131.4 and 141.4. Hoses, syringes and the like can be attached to the fitting 111.4, 121.4, 131.4 and 141.4 in a known manner.

The valve housing 101 further comprises a unifying channel 102.4 into which the valve outlets of the valves 110.4, 120.4, 130.4 and 140.4 open. When, for example, valve 110.4 is open, a fluid can be conducted from the valve inlet 111.4 through the valve 110.4 into the unifying channel 102.4. The unifying channel 102.4 is aligned at right angles to the valve longitudinal directions. The unifying channel 102.4 is configured in the present case as a bore in the valve housing 101.4. The bore is closed on one side by a closure 103.4. At the opposite end, the unifying channel 102.4 encompasses a pressure sensor 150.4, with which the pressure in the unifying channel 102.4 can be measured. In the process, the pressure sensor 150.4 can be used to monitor the pressure in order to control the valves 110.4, 120.4, 130.4 and 140.4 based on the measured pressure data.

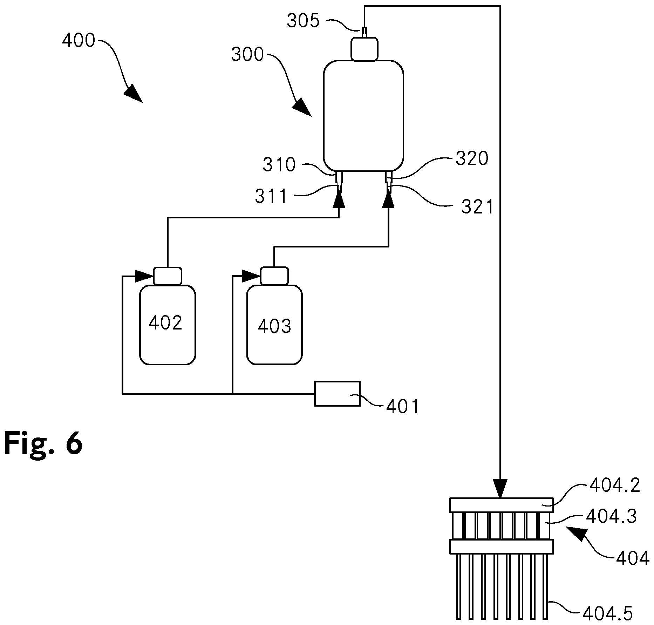

The unifying channel 102.4 is ultimately connected to an output channel 104.4, which in turn is arranged in parallel to valves 110.4, 120.4, 130.4 and 140.4. The output channel 104.4 finally opens into a fitting 105.4, via which the multi-way valve device can be connected to a dispensing head, an injection needle or another multi-way valve device. The following figures 6 to10 show applications of the multi-way valve device in systems. Even if the pressure sensor or the pressure measurement is not explicitly mentioned, it should be understood that these are present and that the valves are controlled on the basis of pressure measurements.

Figure 6 shows a schematic representation of a first system 400 using the third embodiment of the multi-way valve device 300 with two valves.

The system includes two fluid containers 402 and 403, wherein the fluid container 402 contains a cleaning fluid and the fluid container 403 contains a medium that is used in the application of the system. The two fluid containers 402 and 403 are pressurised with compressed air from the compressed air source 401, so that the fluids in the two fluid containers 402 and 403 are under constant pressure. The two fluid containers 402 and 403 are each connected to one of two valve inlets 311, 321 of the two valves 310, 320 of the multi-way valve device 300. The multi-way valve device 300 includes, analogous to the multi-way valve device 100, a pressure sensor in the unifying channel (not shown), which is used to monitor the pressure and control the valves 310, 320 based on the pressure data. The multi-way valve device 300 comprises an output channel connected to the unifying channel (not shown), which finally opens into a fitting 305. Via the fitting 305, the multi-way valve device 300 is ultimately connected to a dispensing head 404, which in this case comprises 8 needles for dispensing the medium from the fluid container 403.

The dispensing head 404 comprises a distributor head 404.2, via which the supply from the fitting 305 is distributed to eight valves 404.3, each of which is connected to a needle 404.5. In the present embodiment, all needles can be controlled separately and individually using valves 404.3. The pressure data from the pressure sensor are used to correct the opening times for metering via the eight valves 404.3.

During the process, it is now possible to switch between a cleaning process and a dosing process via the control of the multi-way valve device 300. In the cleaning process, the first step is to flush cleaning fluid through valve 310. The cleaning fluid thus passes through the unifying channel and exits at dispensing head 404. This allows the system to be flushed with the cleaning fluid. After the rinsing process, the valve can be rinsed with the medium from the fluid container 403, or the dead volume of the dispensing head can be filled with it. The first system 400 is now cleaned and ready to be used to dispense the medium in the dosing process. During the dosing process, valve 310 remains closed and dosing is carried out via valve 320. The control is carried out via pressure data of the unifying channel and is designed as a time control, i.e. valve 320 remains open for a period of time which, according to the measured pressure data, leads to the dosing of the desired volume. The duration can also be adjusted during the dosing process if necessary due to changing pressure data.

Figure 7a shows a schematic representation of a second system 500 using the first embodiment of the multi-way valve 100 (see figure 1).

The multi-way valve device 100 comprises four valve inlets 111, 121, 131, 141 for the four valves 110, 120, 130, 140. The first valve inlet 111 is connected to a first fluid container 503 in which a first cleaning fluid is present. The second valve inlet 121 is connected to a second fluid container 504, in which a second cleaning fluid is present. Furthermore, a compressed air source 501 is provided, with which the two fluid containers 503 and 504 are supplied with a constant pressure. The third valve inlet 131 is also connected to the compressed air source 501. Finally, a vacuum source 502 is provided, which is connected to the fourth valve inlet 141 via a fluid trap 506. This allows the multi-way valve device 100 to switch between two cleaning fluids and between vacuum and compressed air. The output channel of the multi-way valve device 100 opens into a fitting 105, which is connected to a suction head 505 comprising 8 suction needles.

The suction/dispensing head 505 includes a distributor head 505.2, via which the supply from the fitting 105 is distributed to eight valves 505.3, each of which is connected to a needle 505.5. This allows the dosage of fluids to be individually controlled via the 505.3 valves.

The suction/dispensing head 505 can also be used to aspirate fluids in the process, in particular, for example, in an ELISA process. To do this, the valve 140 of the multi-way valve device 100 is opened and the suction head 505 is connected to the vacuum source 502 via the fluid trap 506. After the fluid has been sucked up, it flows through the valve 140, out of the valve inlet 141 into the fluid trap 506. In an alternative process, the fluid can be blown out of the suction needles of the suction head 505 for disposal by switching the valves (close valve 140, open valve 130).

To clean the suction head 505, different cleaning fluids can be fed from the fluid containers 503 and 504 through the suction head 505 via valves 110 and 120. Before cleaning or after cleaning, the suction head 505 can optionally also be blown out via valve 130 in order to free the suction head 505 from adhering residues in the first case or to dry it in the second case.

Figure 7b shows a schematic representation of the second system according to figure 7a, wherein instead of a fluid trap, a volume for storing a fluid is provided in the periphery of the dispensing unit.

The embodiment of the second system is essentially the same as that of figure 7a. However, the present system does not include a fluid trap. This means that the aspirated fluid is not collected in a separate container, but instead can be expelled again, as described above. To this end, the present embodiment of the second system comprises a suction/dispensing head 505.1. This includes a distributor head 505.2, via which the supply from the fitting 105 is distributed to eight valves 505.3, each of which is connected to a needle 505.5. A loop 505.4 is provided between the needle 505.5 and the valves 505.3, in which a fluid can be temporarily stored. This means that a fluid can be sucked up with the suction head 505.1 and stored temporarily in the loop 505.4 so that it can be expelled again later. In the present case, all needles can be controlled separately and individually using valves 505.3.

In a further embodiment, the valves 505.3 can be dispensed with, or exactly one valve can be provided, via which all needles are controlled in parallel. Furthermore, the volume for the intermediate storage can also be arranged between the distributor head 505.2 and the fitting 105, so that exactly one intermediate storage is provided for all needles. The intermediate storage does not necessarily have to be present as a loop, but can also be provided in a different way, as a hose, in the valves or elsewhere.

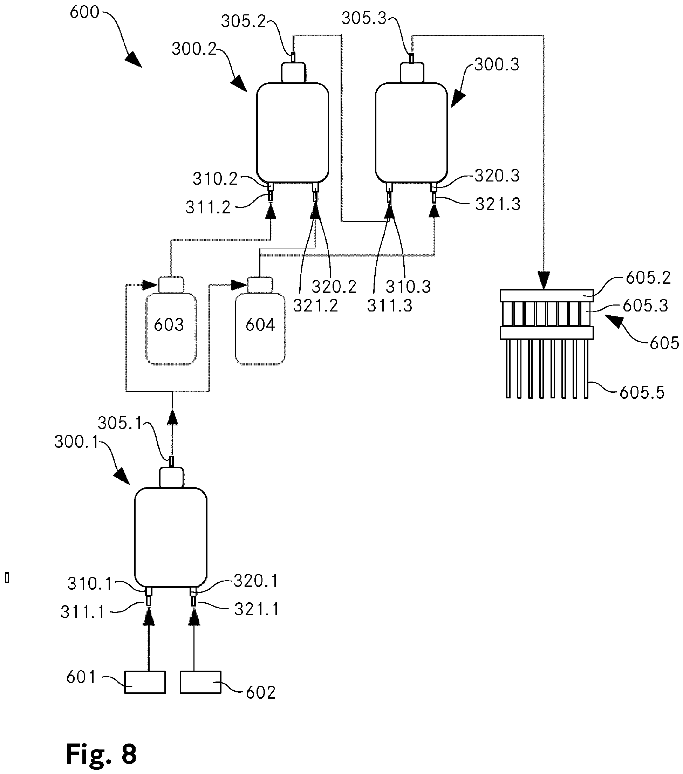

Figure 8 shows a schematic representation of a third system 600 using three multi-way valves 300.1, 300.2 and 300.3 of the third embodiment.

A first multi-way valve 300.1 is connected to a compressed air source 601 via a first valve inlet 311.1 of the first valve 310.1 and to a vacuum source 602 via a second valve inlet 321.1 of the second valve 320.1. The output channel of the multi-way valve device 300.1 opens into a fitting 305.1, which is connected via a branched line to a first fluid container 603, comprising a cleaning fluid, and a second fluid container 604, comprising a medium. With this arrangement, either a vacuum or a pressure can be optionally generated in the fluid containers 603 and 604. At pressure, the medium can be metered or the arrangement can be rinsed with the cleaning solution. At vacuum, after metering has been completed, excess medium can be drawn back into the container 604. This achieves a particularly economical process.

In the present system 600, the first fluid container 603 is connected to a first valve inlet 311.2 of the first valve 310.2 of a second multi-way valve device 300.2. The second fluid container 604 is connected, on the one hand, to the second valve inlet 321.2 of the second valve 320.2 of the second multi-way valve device 300.2 and, on the other hand, to a second valve inlet 321.3 of a second valve 320.3 of a third multi-way valve device 300.3. The output channel of the multi-way valve 300.2 opens into a fitting 305.2, which is connected to the first valve inlet 311.3 of the first valve of the third multiway valve 300.3. The output channel of the multi-way valve 300.3 opens into a fitting 305.3, which is connected to a suction/dispensing head 605 comprising 8 needles.

The suction/dispensing head 605 includes a distributor head 605.2, via which the supply from the fitting 305.3 is distributed to eight valves 605.3, each of which is connected to a needle 605.5. This allows the dosage of fluids to be individually controlled via the 605.3 valves.

In the application, the cleaning fluid is first pumped from the fluid container 603 through the multiway valve device 300.2 and 300.3 for rinsing. Then the medium is pumped through the multi-way valve device 300.2 and 300.3. When the arrangement is used to dispense the medium, the medium is pumped directly via multi-way valve 300.3 to the suction/dispensing head 605. This largely prevents contamination with the cleaning fluid during dispensing, especially since the dead volume from the unifying channel of the multi-way valve 300.2 is completely filled with the medium.

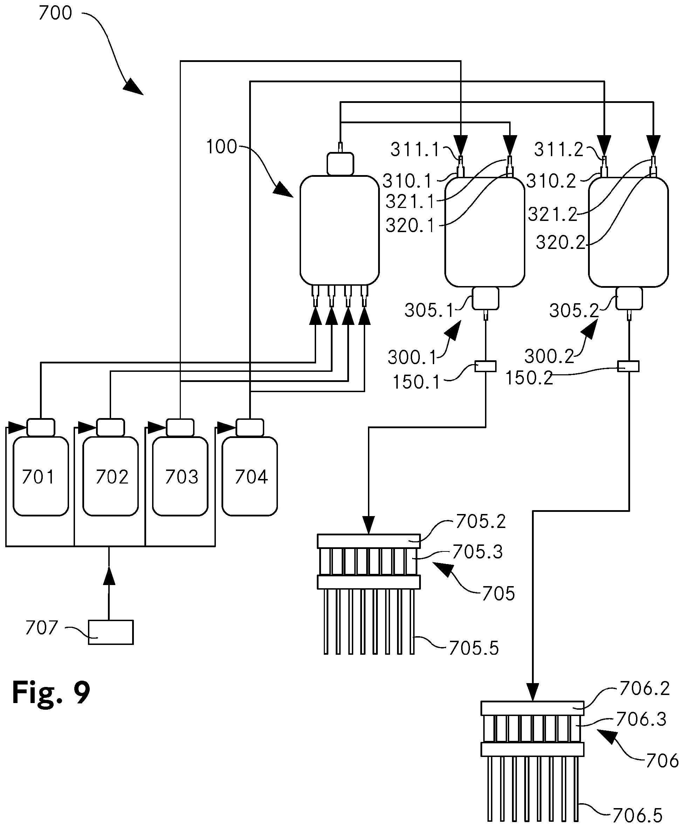

Figure 9 shows a schematic representation of a fourth system 700 using one multi-way valve 100 of the first embodiment and two multi-way valves 300.1 and 300.2 of the third embodiment.

The fourth system 700 comprises four fluid containers 701, 702, 703 and 704, which are pressurised by a compressed air source 707. The two fluid containers 701 and 702 each contain a cleaning fluid, while the other two fluid containers 703 and 704 each contain a medium. The four fluid containers 701 - 704 are each connected to a valve inlet 111, 121, 131, 141 of the valves 110, 120, 130, 140 of the valve 100 of the first embodiment. The output channel of the multi-way valve device 100 opens into a fitting 105, which is connected to a second valve inlet 321.1 of the second valve 320.1 of the first multi-way valve device 300.1. The output channel of the multi-way valve device 100 is further connected via a branch to a second valve inlet 321.2 of the second valve 320.2 of the second multiway valve device 300.2. The fluid container 703 is further connected to a first valve inlet 311.1 of the first valve 310.1 of the first multi-way valve device 300.1. The fluid container 704 is further connected to a first valve inlet 311.2 of the first valve 310.1 of the second multi-way valve 300.2.

The output channel of the multi-way valve 300.1 opens into a fitting 305.1, which is connected to a first dispensing head 705. The output channel of the multi-way valve device 300.2 opens into a fitting 305.2, which is connected to a second dispensing head 706. The dispensing heads 705 and 706 are identical in construction to the dispensing head 605 of Figure 8.

With the system 700, three multi-way valve devices can be used to control two dispensing heads 705 and 706 and to operate with two different cleaning fluids and two different media.

In the present embodiment, the pressure sensor 150.1, 150.2 is not an integral component of the multi-way valve device 311.1 or 311.2, but the pressure sensor 150. 1, 150.2 is arranged in the fluid line between the fitting 305.1 and the dispensing head 705 or between the fitting 305.2 and the dispensing head 706.

The system 700 is cleaned in the same way as the system 600. The arrangement of the multi-way valves in the system 700 also largely prevents contamination with the cleaning fluid during dosing, since the dead volume from the unifying channel of the multi-way valve 100 can be completely filled with the medium from the fluid container 703 or 704.

Figure 10 shows a schematic representation of a fifth system 800 using three multi-way valves 200.1, 200.2 and 200.3 of the second embodiment.

In the present case, the fifth system 800 is designed for use in a medical application, in particular for the injection of medical fluids via infusion.

The fifth system 800 comprises a fluid container 802 with an infusion solution, in particular a salt solution. The fluid container 802 is connected to a compressed air source 801 so that the fluid container 802 can be kept under constant pressure.

The outlet of the fluid container 802 has a branch, with one fluid flow being directed directly to a pressure sensor 806 and the other fluid flow being connected to the valve inlet 221.1 of the second valve 220.1 of the first multi-way valve unit 200.1. The branching can also be realised by a multi-way valve device with two valves. The first valve 200.1 comprises three further valves, of which only the first valve 210.1 and the third valve 230.1 are visible. The three further valve inlets are not occupied in the present case, but could be provided with syringes, via which a drug is admixed to the infusion solution, controlled by the corresponding valves. The output channel of the multi-way valve device 200.1 opens into a Luer lock connection 205.1, which is connected to the valve inlet 221.2 of the second valve 220.2 of the second multi-way valve device 200.2.

The second valve 200.2 comprises three further valves, of which only the first valve 210.2 and the third valve 230.2 are visible. Two of these three further valve inlets are not occupied in the present case. A syringe 803 is connected to the valve inlet of the third valve 230.2 via a Luer lock connection. Syringe 803 contains a medication solution that can be added to the infusion solution via the second multi-way valve device 200.2. The output channel of the multi-way valve device 200.2 opens into a Luer lock connection 205.2, which is connected to the valve inlet 221.3 of the second valve 220.3 of the third multi-way valve device 200.3.

The third valve 200.3 in turn comprises three further valves, of which only the first valve 210.3 and the third valve 230.3 are visible. Two of these three further valve inlets are not occupied in the present case. A syringe 804 is connected to the valve inlet of the third valve 230.3 via a Luer lock connection. Syringe 804 contains a further medication solution that can be added to the infusion solution via the third multi-way valve device 200.3.

The output channel of the third multi-way valve device 200.3 opens into a Luer lock connection 205.3. A line is connected to the Luer lock connection 205.3, which can ultimately be connected to a patient's injection cannula. The branch that includes the pressure sensor 806, via which the dosage is controlled with the valves of the multi-way valve device, also opens into this line. Furthermore, a flow sensor/air bubble sensor 805 is coupled to this line. The air bubble sensor is used to prevent the injection of air bubbles, while the flow sensor can be used to monitor the function of the system (e.g. to determine whether there is a blockage in the system). In the illustration in Figure 10, the injection solution is pumped against gravity through the multi-way valve. This simplifies the connection of the syringe and also reduces the risk of air bubbles entering the system.

It is clear to those skilled in the art that in system 800 the serial arrangement can also be carried out with two multi-way valve devices or more than three multi-way valve devices. The second embodiment of the multi-way valve device is selected for connecting the syringe due to the optimal ergonomics.

The invention provides an multi-way valve device as well as systems comprising one or more multiway valve device, which are particularly precisely controllable and are used in a wide range of applications, in particular for laboratory purposes as well as in medical applications for the injection of drugs into infusion systems.

## Claims

1. Multi-way valve device (100, 200, 300) comprising a valve housing (101) accommodating at least a first valve (110, 210, 310) and a second valve, wherein the first valve (110, 210, 310) comprises a first valve inlet and a first valve outlet and the second valve comprises a second valve inlet and a second valve outlet, **characterized in that** the valve housing (101) comprises a unifying channel (102), wherein the first outlet and the second outlet lead into the unifying channel (102).

2. Multi-way valve device (100, 200, 300) according to claim 1, **characterized by** a sensor, in particular a pressure sensor (150), coupled to the unifying channel (102).

3. Multi-way valve device (100, 200, 300) according to claim 2, **characterized in that** the sensor is arranged lateral to the unifying channel (102), preferably at right angles to a main flow direction of the multi-way valve device (100, 200, 300).

4. Multi-way valve device (100, 200, 300) according to one of claims 1 to 3, **characterized in that** the first valve (110, 210, 310) and/or the second valve are designed as solenoid valves.

5. Multi-way valve device (100, 200, 300) according to one of claims 1 to 4, **characterized in that** the first valve (110, 210, 310) and the second valve are arranged in parallel.

6. Multi-way valve device (100, 200, 300) according to one of claims 1 to 5, **characterized in that** the first fluid outlet and/or the second fluid outlet are connected to the unifying channel (102) via a rigid fluid line.

7. Multi-way valve device (100, 200, 300) according to claim 6, **characterized in that** the unifying channel (102) is formed as bores in the valve housing (101).

8. Multi-way valve device (100, 200, 300) according to one of claims 1 to 7, **characterized in that** the first valve (110, 210, 310) and the second valve are designed as separate units from the valve housing (101), which can be connected to the valve housing (101) in particular via detachable connections.

9. Multi-way valve device (100, 200, 300) according to one of claims 1 to 8, **characterized in that** at least the first valve inlet comprises a first fitting for directly connecting the first valve inlet with a syringe, in particular a first Luer lock connection and, preferably, the unifying channel (102) comprising a second fitting, in particular a second Luer lock connection.

10. Arrangement comprising a first multi-way valve device (100) and a second multi-way valve device (300), in particular a first multi-way valve device (100) and a second multi-way valve device (300) according to one of the preceding claims, **characterised in that** the first multi-way valve device (100) and the second multi-way valve device (300) are arranged in series, whereby the second multi-way valve device (300) is arranged downstream of the first multi-way valve device (100).

11. Arrangement according to claim 10, **characterized in that** a unifying channel (102) of the first multi-way valve device (100) is in fluid communication with an inlet of a first valve (310) of the second multi-way valve device (300) and wherein an inlet of a first valve (110) of the first multiway valve device (100) is directly connected to a source of a cleaning fluid and an inlet of a second valve (120) of the first multi-way valve device (100) is directly connected to a source of a dosing medium, and wherein an inlet of a second valve (320) of the second multi-way valve device (300) is directly connected to the source of the dosing medium.

12. Arrangement according to claim 11, **characterized in that** a unifying channel of the second multiway valve device (300) is in fluid communication with a dispensing head (705), wherein the dispensing head (705) preferably comprises at least two dispensing needles for dispensing the dosing medium.

13. Arrangement according to claim 10, **characterized in that** a unifying channel of the first multiway valve device is in fluid communication with an inlet of a first valve of the second multi-way valve device and wherein an inlet of a first valve of the first multi-way valve device is directly connected to a source of a first medical fluid and an inlet of a second valve of the first multiway valve device is directly connected to a source of a infusion fluid, and wherein an inlet of a second valve of the second multi-way valve device is directly connected to the source of a second medical fluid.

14. Arrangement according to claim 13, **characterized in that** the first medical fluid is contained in a first syringe, which is connected directly to the inlet of the first valve of the first multi-way valve device, preferably via a Luer lock connection and that, preferably, the second medical fluid is contained in a second syringe, which is connected directly to the inlet of the first valve of the second multi-way valve device, preferably via a Luer lock connection, and wherein preferably the first syringe and the second syringe each comprise a plunger, which are pressurised at a constant pressure during operation.

15. Arrangement according to claim 13 or 14, **characterized in that** a unifying channel of the second multi-way valve device is in fluid communication with an medical injection needle.

16. Arrangement according to claim 15, **characterized by** a flow sensor and/or an air bubble sensor coupled to the unifying channel of the first multi-way valve device.

17. Method for cleaning an arrangement according to claim 10, wherein a cleaning fluid is successively fed through the first multi-way valve device (100) and the second multi-way valve device (300) and, in particular, a dosing medium or an infusion fluid is successively fed through the first multi-way valve device (100) and the second multi-way valve device (300) and the dosing medium or the infusion fluid, is fed directly through the second multi-way valve device (300).

18. Method for controlling a multi-way valve device (100, 200, 300) according to claim 1, **characterised by** measuring data, in particular pressure data of a dosing medium in the unifying channel (102), and wherein a dosing volume of the dosing medium is controlled on the basis of the data.

19. Method according to claim 18, wherein a fluid inlet of a first valve of the multi-way valve device (100, 200, 300) is pressurised with the dosing medium at constant pressure.

20. Use of a multi-way valve device (100, 200, 300) according to one of claims 1 to 9 for controlling a flow of a fluid in a laboratory application.

21. Use of a multi-way valve device (100, 200, 300) according to one of claims 1 to 9 for controlling a flow of an infusion fluid in a medical application.
